(19) Europäisches Patentamt
European Patent Office
Office européen des brevets

(11) Publication number: **0 455 427 A1**

# (12) EUROPEAN PATENT APPLICATION

(21) Application number: **91303761.0**

(22) Date of filing: **25.04.91**

(51) Int. Cl.[5]: **C07D 498/18**, C07H 19/01, A61K 31/435, // (C07D498/18, 311:00, 273:00, 221:00)

(30) Priority: **30.04.90 US 516598**

(43) Date of publication of application: **06.11.91 Bulletin 91/45**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Goulet, Mark T.**
**719 Hanford Place**
**Westfield, NJ 07090 (US)**
Inventor: **Wyvratt, Matthew J.**
**1130 Puddingstone Road**
**Mountainside, NJ 07092 (US)**
Inventor: **Hodkey, Derek W.**
**48 Maple Court**
**Highland Park, NJ 08904 (US)**

(74) Representative: **Thompson, John Dr. et al**
**Merck & Co., Inc. European Patent Department Terlings Park Eastwick Road**
**Harlow, Essex CM20 2QR (GB)**

## (54) Deoxymacrolide derivatives having immunosuppressive activity.

(57) Deoxymacrolide derivatives of the general structural Formula I:

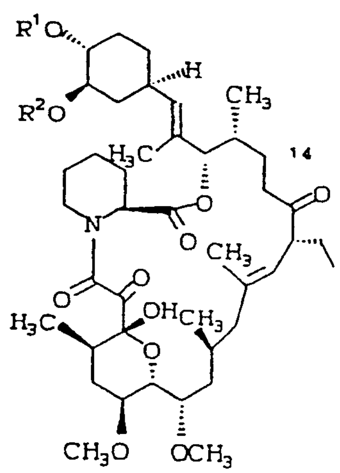

I

have been prepared from (a) suitable precursor(s) by selective removal of the hydroxyl at C-14. These macrolide immunosuppressants are useful in a human host for the treatment of autoimmune diseases, infectious diseases and/or the prevention of rejection of foreign organ transplants. In addition, these macrolide immunosuppressants are useful in the topical treatment of inflammatory and hyperproliferative skin diseases and cutaneous manifestations of immunologically-mediated illnesses.

EP 0 455 427 A1

SUMMARY OF THE INVENTION

The present invention is related to deoxymacrolides which are useful in a human host for the treatment of autoimmune diseases (such as juvenile-onset diabetes mellitus, multiple sclerosis and rheumatoid arthritis), infectious diseases and/or the prevention of rejection of foreign organ transplants, e.g. bone marrow and heart transplants and are also useful in the topical treatment of inflammatory and hyperproliferative skin diseases and cutaneous manifestations of immunologically-mediated illnesses such as: psoriasis, atopical dermatitiis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus or Alopecia areata. More particularly, this invention relates to compounds of the general structural

Formula I:

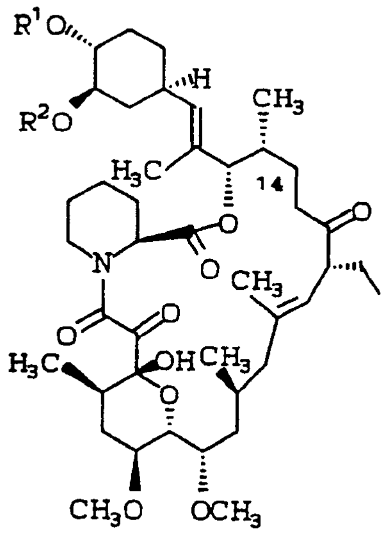

I

wherein
$R^1$ and $R^2$ are, independently, hydrogen or a hydroxyl protecting group.

This invention also relates to pharmaceutical compositions containing the compounds and to a method of use of the present compounds and other agents for the treatment of autoimmune diseases, infectious diseases and/or the rejection of foreign organ transplants.

BRIEF DESCRIPTION OF DISCLOSURES IN THE ART

Fujisawa United States, European and Japanese patents (U.S. Patent No. 4,894,366, (EPO Publication No. 0,184,162 and PBJ Disclosure 63-17884) and publications (J. Am. Chem. Soc., 1987, 109, 5031 and J. Antibiotics, 1987, 40, 1249) disclose 17-allyl-1,14-dihydroxy-12-[2'-(4"-hydroxy-3"-methoxycyclohexyl)-1'-methylvinyl]-23,25-dimethoxy-13,19,21,27-tetramethyl-11,28-dioxa-4- azatricyclo[22.3.1.0$^{4,9}$]octacos-18-ene-2,3,10,16-tetraone (FR-900506), 17-ethyl-1,14-dihydroxy-12-[2'-(4"-hydroxy-3"-methoxycyclohexyl)-1'-methylvinyl]-23,25-dimethoxy-13,19,21,27-tetramethyl-11,28-dioxa-4- azatricyclo[22.3.1.0$^{4,9}$]octacos-18-ene-2,3,10,16-tetraone (FR-900520) and related compounds which are the starting materials for the preparation of the compounds described. The synthetic preparation of the aforementioned starting material (FR-900506) has recently been reported (J. AM. Chem. Soc., 1989, III, 1157). A Fisons European patent (EPO Publication No. 0,323,042) discloses various derivatives of FR-900506, FR-900520 and related compounds. A Sandoz European patent (EPO Publication No. 0,315,978) discloses the use of FR-900506 and related compounds in the topical treatment of inflammatory and hyperproliferative skin diseases and of cutaneous manifestations of immunologically-mediated illness.

## BACKGROUND OF THE IVENTION

Immunoregulatory abnormalities have been shown to exist in a wide variety of "autoimmune" and chronic inflammatory diseases, including systemic lupus erythematosis, chronic rheumatoid arthritis, type I diabetes mellitus, inflammatory bowel disease, biliary cirrhosis, uveitis, multiple sclerosis and pemphigoid, sarcoidosis, psoriasis, ichthyosis, and other disorders such as Chrons disease, ulcerative colitis, bullous Graves ophthalomopathy. Although the underlying pathogenesis of each of these conditions may be quite different, they have in common the appearance of a variety of autoantibodies and self-reactive lymphocytes. Such self-reactivity may be due, in part, to a loss of the homeostatic controls under which the normal immune system operates.

Similarly, following a bone-marrow or an organ transplantation, the host lymphocytes recognize the foreign tissue antigens and begin to produce antibodies which lead to graft rejection.

One end result of an autoimmune or a rejection process is tissue destruction caused by inflammatory cells and the mediators they release. Antiflammatory agents such as NSAID's and corticosteroids act principally by blocking the effect or secretion of these mediators but do nothing to modify the immunologic basis of the disease. On the other hand, cytotoxic agents such as cyclophosphamide, act in such a nonspecific fashion that both the normal and autoimmune responses are shut off. Indeed, patients treated with such nonspecific immunosuppressive agents are as likely to succumb from infection as they are from their autoimmune disease.

Cyclosporin A which was licensed by the US FDA in I983 is currently the leading drug used to prevent rejection of transplanted organs. The drug acts by inhibiting the body's immune system from mobilizing its vast arsenal of natural protecting agents to reject the transplant's foreign protein. Though cyclosporin A is effective in fighting transplant rejection, it is nephrotoxic and is known to cause several undesirable side effects including kidney failure, abnormal liver function and gastro-intestinal discomfort.

Newer, safer drugs exhibiting less side effects are constantly being searched for in the field.

The 23-membered tricyclo-macrolide immunosuppressant, FR-900506,

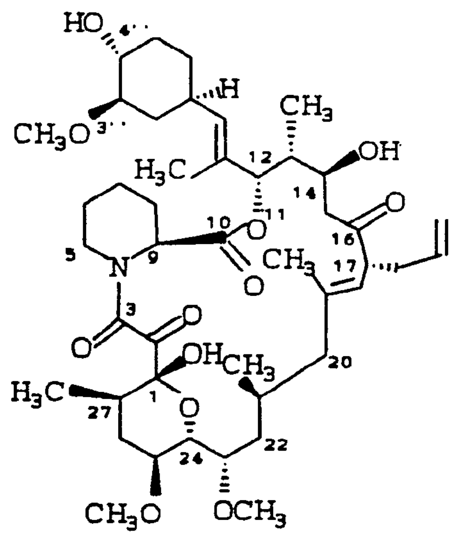

and related compounds which were isolated and characterized by Tanaka, Kuroda, and co-workers at Fujisawa Pharmaceutical Co. in Japan, see J. Am. Chem. Soc., I987, I09, 503I, and U.S. patent No. 4,894,366 (issued Jan. I6, I990), have been shown to possess exceptional immunosuppressive activity. The compound FR-900506 has been reported to be I00 times more effective than cyclosporin in the suppression of in vitro immune systems (J. Antibiotics I987, 40, I256). In addition, these compounds are reputed to possess topical activity in the treatment of inflammatory and hyperproliferative skin diseases and cutaneous manifestations of immunologically-mediated illnessess (EPO Pub. No. 0,3I5,978).

Accordingly, an object of the present invention is to provide new analogs of these tricyclo-macrolides which will (I) restore the balance of the help-and-suppression mechanism of the immune system by acting at an earlier point than the anti-inflammatory agents and (2) induce specific long-term transplantation tolerance through a suppressor cell circuit without increasing the body's susceptibility to infection.

An additional object of the present invention is to provide analogs of these tricyclo-macrolides which possess topical activity in the treatment of inflammatory and hyperproliferative skin diseases and cutaneous manifestations of immunologically-mediated illnesses.

Another object of the present invention is to provide pharmaceutical compositions for administering to a patient in need of the treatment one or more of the active immunosuppressive agents of the present invention.

Still a further object of this invention is to provide a method of controlling graft rejection, autoimmune and chronic inflammatory dieases by administering a sufficient amount of one or more of the novel immunosuppressive agents in a mammalian species in need of such treatment.

Finally, it is the object of this invention to provide processes for the preparation of the active compounds of the present invention.

## DETAILED DESCRIPTION OF THE INVENTION

### A. Scope of the invention

The novel compounds of this invention have structural Formula I:

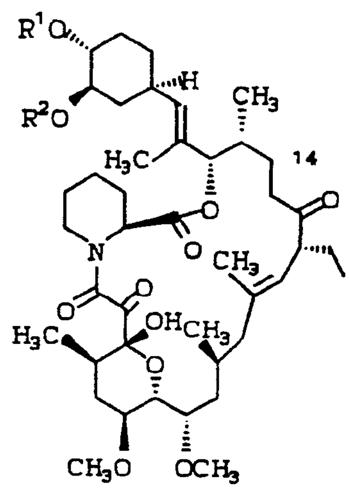

I

wherein:

$R^1$ and $R^2$ are, independently, hydrogen or a hydroxyl protecting group.

The compounds of the present invention have asymmetric centers and this invention includes all of the possible isomers and mixtures thereof.

In the present invention it is preferred that in compounds of Formula I:

$R^1$ is hydrogen; and

$R^2$ is hydrogen.

The preferred compound of the present invention is the compound:

I7-ethyl-I-hydroxy-I2-[2'-(3",4"-dihydroxy-cyclohexyl)-I'-methylvinyl]-23,25-dimethoxy-I3,I9,2I,27-tetra methyl-II,28-dioxa-4- azatricyclo[22.3.I.$0^{4,9}$]octacos-I8-ene-2,3,I0,I6-tetraone.

### B. Preparation of Compounds Within the Scope of the Present Invention

The starting materials for the preparation of the compounds of this invention are represented by Formula II:

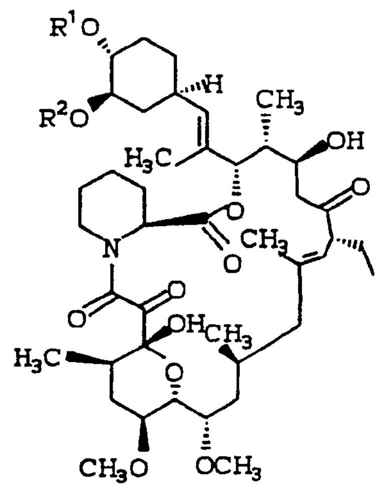

II

wherein:

$R^1$ is hydrogen or a hydroxyl protecting group,

$R^2$ is methyl, hydrogen or a hydroxyl protecting group.

The production of compounds of Formula II wherein $R^1$ is hydrogen and $R^2$ is methyl is well known in the literature (see U.S. Patent No. 4,894,366, issued Jan. 16, 1990; EPO Publication No. 0,184,162; PBJ Disclosure 63-17884; J. Am. Chem. Soc., 1987, 109, 5031; and J. Antibiotics, 1987, 40, 1249). Both biological fermentation and synthetic processes may be found. A synthetic route to compounds of Formula II may be found in J. Am. Chem. Soc., 1989, 111, 1157.

Biological fermentation followed by synthetic modification is presently favored in the art as the method to produce compounds of Formula II. Organisms belonging to the genus Streptomyces such as Streptomyces tsukubaensis, No. 9993 and Streptomyces hygroscopicus, No. 7238 placed in an aqueous nutrient medium will produce the desired compounds in isolable amounts. The nutrient medium contains sources of assimilable carbon and nitrogen, preferably under aerobic conditions. Produced in fermentation is the compound of Formula II where $R^1$ is hydrogen and $R^2$ is methyl. Three related compounds are also produced by fermentation.

A lyophilized sample of the isolated Streptomyces tsukubaensis, No. 9993 was deposited with the Fermentation Research Institute, Agency of Industrial Science and Technology (No. 1-3, Higashi 1-chome, Yatabemachi Tsukuba-gun, Ibaraki Prefecture, Japan) under the deposit number of FERM P-7886 (deposit date: October 5th, 1984), and then converted to Budapest Treaty route of the same depository on October 19, 1985 under the new deposit number of FERM BP-927.

Using the compound produced in fermentation above, the remaining compounds of Formula II may be easily produced. The hydroxyl of C-4", wherein $R^1$ is hydrogen, may be protected by well known methods, for example as disclosed in U.S. patent No. 4,894,366 (issued Jan. 16, 1990) or in EPO Publication 0,323,042. The methyl of $R^2$ as produced may be replaced with hydrogen or demethylated and subsequently protected as desired, if necessary. This demethylation of $R^2$ may be carried out in a fermentation reaction using the compounds of Formula II as a feedstock. For instance, the compound named under Formula II above wherein $R^1$ is hydrogen and $R^2$ is methyl may be demethylated at $R^2$ above using the microorganism Actinoplanacete, ATCC No. 53771 as described in EPO Publication No. 0,349,061. In addition, compounds of Formula II wherein $R^1$ is hydrogen and $R^2$ is hydrogen may be produced directly by fermentation using the mutant microorganism Streptomyces hygroscopicus sup. ascomyceticus, ATCC No. 53855 (being a blocked mutant of Streptomyces hygroscopicus sup. ascomyceticus, ATCC No. 14891) (as taught in U.S. Serial No. 323,653-corresponding to EP-A-0388152, and hereby incorporated by reference).

The term "lower alkyl" used in the specification is intended to mean a straight, cyclic or branched chain of one to six carbon atoms.

Suitable hydroxyl protective groups in the "hydroxyl protecting group" may include those groups well known in the art which are:

1-(lower alkylthio)(lower)alkyl, such as lower alkylthiomethyl (e.g. methylthiomethyl, ethylthiomethyl, propylthiomethyl, isopropylthiomethyl, butylthiomethyl, isobutylthiomethyl, hexylthiomethyl, etc.), and the like, in which the preferred one may be $C_1$ - $C_4$ alkylthiomethyl and the most preferred one may be methylthiomethyl; trisubstituted silyl such as tri(lower)alkylsilyl (e.g. trimethylsilyl, triethylsilyl, tributysilyl, tri-i-propylsilyl, tert-butyl-dimethylsilyl, tri-tert-butylsilyl, etc.), lower alkyldiarylsilyl (e.g. methyl-diphenylsilyl, ethyl-diphenylsilyl, propyl-diphenylsilyl, tert-butyl-diphenylsilyl, etc.), and the like, in which the preferred one may be tri($C_1$ - $C_4$) alkylsilyl and $C_1$ - $C_4$ alkyl-diphenylsilyl, and the most preferred one may be tert-butyl-dimethylsilyl, tri-i-propylsilyl and tert-butyl-diphenylsilyl.

The compound of Formula II wherein $R^1$ is hydrogen and $R^2$ is methyl, related compounds, organisms to produce the same, conditions of fermentation, separation techniques, and chemical modification of the product are fully described in U.S. Patent No. 4,894,366 (issued Jan. 16, 1990). This document is hereby incorporated by reference.

The novel processes for preparing the novel compounds of the present invention are illustrated as follows, wherein $R^1$ and $R^2$ are as defined above unless otherwise indicated.

## REACTION SCHEME A

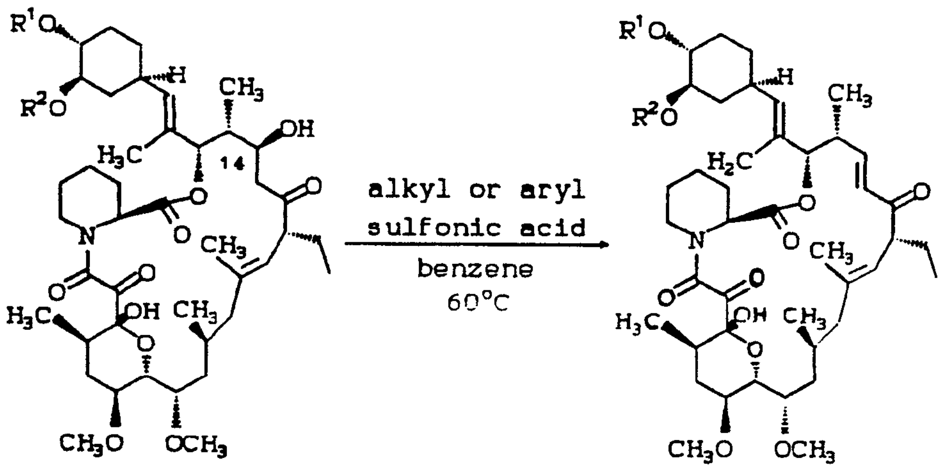


II

III

($R^1$ = H
$R^2$ = H)

As shown in Reaction Scheme A the 14-hydroxy group of the macrolide (II) is eliminated by treatment with p-toluenesulfonic acid, benzenesulfonic acid, methanesulfonic acid, p-nitrobenzenesulfonic acid, p-bromobenzenesulfonic acid, p-chlorobenzenesulfonic acid, or p-methoxybenzenesulfonic acid, or mixtures thereof, in an inert organic solvent such as benzene, or toluene or the like at a temperature of 40°C to solvent reflux temperature, preferably 60°C, for about 0.5 to 6 hours, or a sufficient period of time to eliminate the 14-hydroxy group. Neutralization with an aqueous solution of a weak base such as saturated sodium bicarbonate gives the 14,15-dehydro macrolide (III).

## REACTION SCHEME B

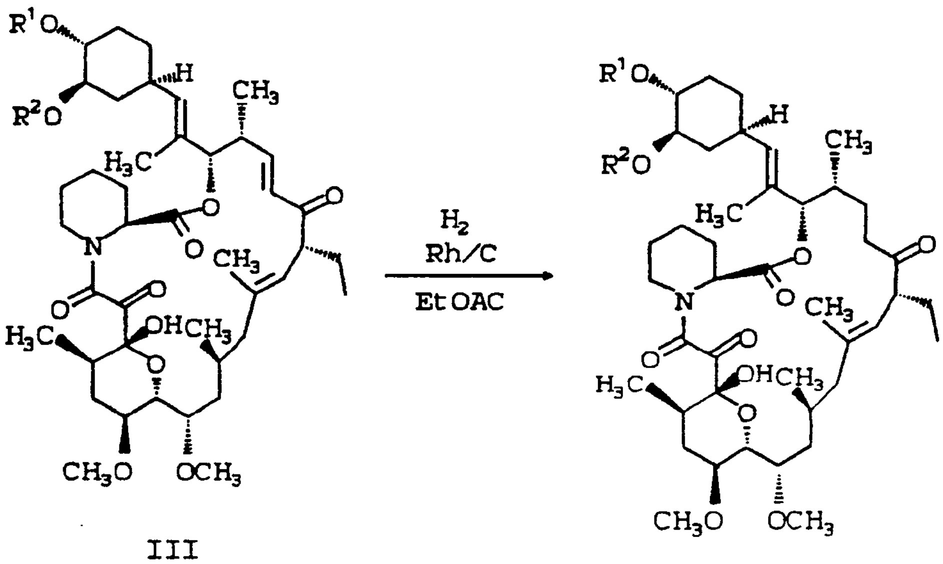


III

($R^1$ = H
$R^2$ = H)

I

As shown in Reaction Scheme B the I4, I5-dehydro macrolide (III) is reduced under an atmosphere of hydrogen in the presence of a noble metal catalyst, such as rhodium on carbon catalyst or rhodium on alumina catalyst, at a pressure of atmospheric pressure to 40 psig, at or near room temperature in an organic solvent such as ethyl acetate or ethanol for about I to 24 hours, or until the requisite amount of hydrogen is absorbed to reduce the I4,I5-olefin and give the I4-deoxymacrolide of Formula I.

Protection of the C-3″ and/or the C-4″ hydroxyl group may be accomplished by methods known in the prior art for compounds of Formula II such as by treatment with: 2,6-lutidine and triisopropylsilyl trifluoromethane sulfonate in a solution of methylene chloride; 2,6-lutidine and t-butyldimethylsilyl trifluoromethanesulfonate in a solution of methylene chloride; pyridine and acetic anhydride in a solution of methylene chloride; pyridine and benzoyl chloride in a solution of dichloromethane; pyridine and p-nitrobenzoyl chloride in a solution of dichloromethane; imidazole and t-butyldiphenylsilyl chloride in a solution of methylene chloride; and the like.

The object compounds of Formula I obtained according to the reactions as explained above can be isolated and purified in a conventional manner, for example, extraction, precipitation, fractional crystallization, recrystallization, chromatography, and the like.

It is to be noted that in the aforementioned reactions and the post-treatment of the reaction mixture therein, the conformer and/or stereo isomer(s) of the object compounds of Formula I due to asymmetric carbon atom(s) or double bond(s) of the starting and object compounds may occasionally be transformed into the other conformer and/or stereoisomer(s), and such conformers and stereoisomers are also included within the scope of the present invention.

In the present invention, compounds with asymmetric centers may occur as racemates, racemic mixtures and as individual diastereomers, with all isomeric forms of the compounds being included in the present invention. These may be prepared by methods such as those disclosed in publications which describe synthetic routes to fragments of the macrolide FR-900506 and the total synthesis of the macrolide FR-900506 itself (Tetrahedron Lett., I988, 29, 277; Tetrahedron Lett., I988, 29, 28I; Tetrahedron Lett., I988, 29, 448I; J. Org. Chem., I989, 54, 9; J. Org. Chem., I989, 54, II; J. Org. Chem., I989, 54, I5; J. Org. Chem., I989, 54, I7; J. Am. Chem. Soc., I989, III, II57).

In addition compounds with carbon-carbon double bonds may occur in cis and trans form with all isomeric forms of the compounds being included in the present invention.

C. Utility of the compounds within the scope of the invention

The compounds of Formula I may be employed as immunosuppressants or antimicrobial compounds by methods and in dosages know in the prior art for compounds of Formula II. These compounds possess pharmacological activity such as immunosuppressive activity, antimicrobial activity, and the like, and therefore are useful for the treatment and prevention of the resistance to transplantation or transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosis, Hashimoto's thyroiditis, multiple sclerosis, myasthenia gravis, type I diabetes, uveitis, etc., and infectious diseases caused by pathogenic microorganisms.

The compounds of Formula I are also useful for treating inflammatory and hyperproliferative skin diseases and cutaneous manifestations of immunologically-mediated illnesses such as: psoriasis, atopical dermatitiis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias or Alopecia areata.

The pharmaceutical compositions of this invention can be, for example, in solid, semisolid or liquid form, which contains one or more of the compounds of the present invention, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For the treatment of these conditions and diseases caused by immmunoirregularity a compound of formula I may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. For applying a composition of this invention to a human, it is preferable to apply it by parenteral or oral administration.

Dosage levels of the compounds of the present invention are of the order from about 0.05 mg to about l00 mg per kilogram of body weight per day, preferably from about 0.5 mg to about l0 mg per kilogram of body weight per day, are useful in the treatment of the above-indicated conditions (from about l mg to about 5 gm per patient per day). In addition, the compounds of the present invention may be administered on an intermittent basis such as on semiweekly, weekly, semimonthly or monthly intervals.

The amount of active ingredient that may be combined with the carrier materials to produce a single dosage form will vary depending upon the host treated and the particular mode of administration. For example, a formulation intended for the oral administration of humans may contain from 0.05 mg to 5 gm of active agent compounded with an appropriate and convenient amount of carrier material which may vary from about 5 to about 95 percent of the total composition. Dosage unit forms will generally contain from about 0.5 mg to about 500 mg of active ingredient.

It will be understood, however, that the specific dose level for any particular patient will depend on upon a variety of factors including the activity of the specific compound employed, the age, body weight, general health, sex, diet, time of administration, route of administration, rate of excretion, drug combination and the severity of the particular disease undergoing therapy.

The following examples are given for the purpose of illustrating the present invention and shall not be construed as being limitations on the scope or spirit of the instant invention.

EXAMPLE I

l7-Ethyl-l-hydroxy-l2-[2′-(3″,4″-dihydroxycyclohexyl)-l′-methylvinyl]-23,25-dimethoxy-l3,l9,2l,27-tetramethyl-ll,28-dioxa-4- azatricyclo[22.3.l.0$^{4,9}$]-octacos-l4,l8-diene-2,3,l0,l6-tetraone

To a stirred solution of l7-ethyl-l,l4-dihydroxy-l2-[2′-(3″,4″-dihydroxycyclohexyl)-l′-methylvinyl]-23,25-dimethoxy-l3,l9,2l,27- tetramethyl-ll,28-dioxa-4-azatricyclo[22.3.l.0$^{4,9}$]octacos-l8-ene-2,3,l0,l6-tetraone (77 mg in 3 ml benzene) was added 6 mg of p-toluenesulfonic acid and the mixture warmed to 60°C in an oil bath. After

25 minutes, the reaction mixture was cooled to room temperature, neutralized by the addition of a saturated aqueous $NaHCO_3$ solution and extracted with ethyl acetate (3 times). The combined organics were washed with saturated NaCl solution, dried over $Na_2SO_4$ and purified by flash chromatography (20% hexanes in ethyl acetate and I% MeOH) to yield 40 mg of the title compound.
MASS: (FAB) 782 (m + Na).
Partial $^1H$ NMR (200 mHz): δ 6.80 (dd, $J_1$ = I6 Hz, J2 = 6 Hz), 6.I6 (dd, $J_1$ = I6 Hz, $J_2$ = I.5 Hz), 4.39 (broad d, J = I4 Hz), 4.26 (broad d, J = 5 Hz), 3.9I (dd, $J_1$ = 8.8 Hz, $J_2$ = 3 Hz).

EXAMPLE 2

I7-Ethyl-I-hydroxy-I2-[2′-(3″,4″-dihydroxycyclohexyl)-I′-methylvinyl]-23,25-dimethoxy-I3,I9,2I,27-tetramethyl-II,28-dioxa-4- azatricyclo[22.3.I.0$^{4,9}$]-octacos-I8-ene-2,3,I0,I6-tetraone

To a solution of I7-ethyl-I-hydroxy-I2-[2′-(3″,4″-dihydroxycyclohexyl)-I′-methylvinyl]-23,25-dimethoxy-I3,I9,2I,27-tetramethyl-II,28-dioxa-4-azatricyclo[22.3.I.0$^{4,9}$]octacos-I4,I8-diene-2,3,I0,I6-tetraone (40 mg in I.5 ml ethyl acetate) was added 3 mg of 5% Rh/Carbon catalyst. The reaction flask was fitted with a hydrogen balloon, evacuated and recharged with hydrogen gas (3 times). After 45 minutes, the mixture was filtered over Celite, concentrated and purified by flash chromatography ($CH_2Cl_2$: MeOH: Hexane (I0:I:2)). to yield 33 mg of the title compound.
MASS: (FAB) 768 (m + Li).
Partial $^1H$ NMR (200 mHz): δ 4.55 (broad d, J = 5 Hz), 4.39 (broad d, J = I4 Hz), 3.86 (dd, $J_1$ = 8.8 Hz, $J_2$ = 3 Hz).

EXAMPLE 3

T-Cell Proliferation Assay

I. Sample Prpearation

The compounds to be assayed were dissolved in absolute ethanol at I mg/ml.

2. Assay

Spleens from C57Bl/6 mice were taken under sterile conditions and gently dissociated in ice-cold RPMI I640 culture medium (GIBC) (Grand Island, N.Y.), supplemented with I0% heat-inactivated fetal calf serum (GIBO). Cells were pelleted by centrifugation at I500 rpm for 8 minutes. Contaminating red cells were removed by treating the pellet with ammonium chloride lysing buffer (GIBO) for 2 minutes at 4°C. Cold medium was added and cells were again centrifuged at I500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 25°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for I hour. Non-adherent T lymphocytes were eluted from the columns with warm culture medium and the cell suspensions were spun as above.

Purified T lymphocytes were resuspended at 2.5 x $I0^5$ cells/ml in complete culture medium composed of RPMI I640 medium with I0% heat-inactivated fetal calf serum, I00 mM glutamine, I mM sodium pyruvate, 2 x $I0^{-5}$ M 2-mercaptoethanol and 50 μg/ml gentamycin. Ionomycin was added at 250 ng/ml and PMA at I0 ng/ml. The cell suspension was immediately distributed into 96 well flat-bottom microculture plates (Costar) at 200 μl/well. The various dilutions of the compound to be tested were then added in triplicate wells at 20 μl/well. The compound I7-allyl-I,I4-dihydroxy-I2-[2′-(4″-hydroxy-3-methoxycyclohexyl)-I′-methylvinyl]-23,25-dimethoxy-I3,I9,2I,27-tetramethyl-II,28-dioxa-4- aza-tricyclo[22.3.I.0$^{4,9}$]octacos-I8-ene-2,3,I0,I6-tetraone was used as a standard. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 μCi/well of tritiated thymidine (NEN, Camgridge, MA). After another 4 hours of incubation, cultures were harvested on glass fiber filters using a multiple sample harvester. Radio-activity of filter discs corresponding to individual wells was measured by standard liquid scintillation counting methods (Betacounter). Mean counts per minute of replicate wells were calculated and the results expressed as concentration of compound required to inhibit tritiated thymidine uptake of T-cells by 50%.

A compound was tested according to the previous procedure. The concentration of compound required to inhibit the proliferation of T-cells by 50% was measured, and the results were as follows:

| Example No. Of Product Compound | $IC_{50}$(M) |
|---|---|
| 2 | < 1 x $10^{-6}$ |

The results of this assay are representative of the intrinsic immunosuppressive activity of the compounds of the present invention.

While the foregoing specification teaches the principles of the present invention, with examples provided for the purpose of illustration, it will be understood that the practice of the invention encompasses all of the casual variations, adaptations, modifications, deletions, or additions of procedures and protocols described herein, as come within the scope of the following claims and its equivalents.

## Claims

1. A compound of formula I:

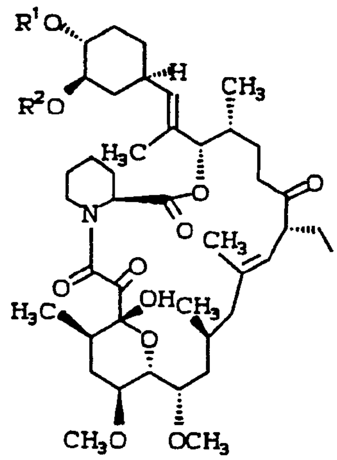

I

wherein:
$R^1$ and $R^2$ are, independently, hydrogen or a pharmaceutically acceptable hydroxyl protecting group.

2. The compound of Claim I wherein:
$R^1$ and $R^2$ are, independently, hydrogen or a pharmaceutically acceptable hydroxyl protecting group selected from I-(lower alkylthio)(lower)alkyl, tri(lower)alkylsilyl, and loweralkyl-diphenylsilyl.

3. The compound of Claim I wherein:
$R^1$ is hydrogen; and
$R^2$ is hydrogen.

4. The compound which is I7-ethyl-I-hydroxy-I2-[2'-(3",4"-dihydroxycyclohexyl)-I'-methylvinyl]-23,25-dimethoxy-I3,I9,2I,27-tetramethyl-II,28-dioxa-4- azatricyclo[22.3.I.$0^{4,9}$]octacos-I8-ene-2,3,I0,I6-tetraone.

5. A pharmaceutical composition for the treatment of immunoregulatory disorders or diseases comprising a pharmaceutical carrier and a therapeutically effective amount of a compound of formula I, according to Claim I.

6. A pharmaceutical composition for the topical treatment of inflammatory and hyperproliferative skin diseases and or cutaneous manifestations of immunologically-mediated illnesses comprising a pharmaceutical carrier and a therapeutically effective amount of a compound of formula I, according to Claim I.

7. The use of a compound of formula I according to Claim I for the manufacture of a medicament for the treatment of immunoregulatory disorders or diseases.

8. The use of a compound of formula I according to Claim I for the manufacture of a medicament for the topical treatment of inflammatory and hyperproliferative skin diseases and or cutaneous manifestations of immunologically-mediated illnesses.

9. A process for the production of a compound of formula I:

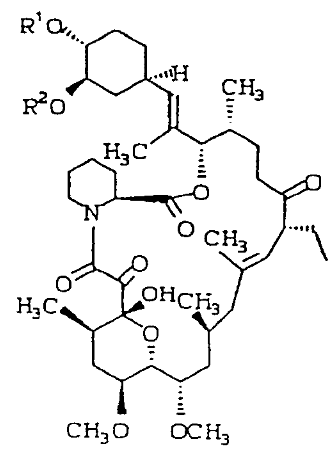

I

wherein:
$R^1$ and $R^2$ are, independently, hydrogen or a hydroxyl protecting group which comprises:
a) contacting a compound of formula IIa

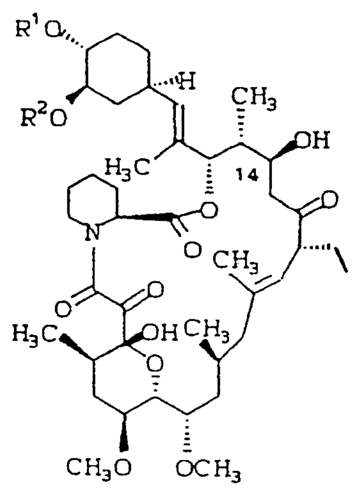

IIa

wherein:
$R^1$ and $R^2$ are, independently, hydrogen or a hydroxyl protecting group;

with p-toluenesulfonic acid, methanesulfonic acid, benzenesulfonic acid, p-nitrobenzenesulfonic acid, p-bromobenzenesulfonic acid, p-chlorobenzenesulfonic acid, or p-methoxybenzenesulfonic acid, or mixtures thereof in an inert organic solvent at a temperature between 40°C and solvent reflux temperature for a sufficient time to eliminate the l4-hydroxyl group;
b) contacting the product of step (a) with a rhodium on carbon catalyst or a rhodium on alumina catalyst in an inert organic solvent at or near room temperature under a hydrogen gas pressure of about atmospheric pressure to 40 psig for a sufficient time to form a compound of Formula I.

European Patent Office

# EUROPEAN SEARCH REPORT

Application Number

EP 91 30 3761

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P<br>X<br><br>A | EP-A-0413532 (FISONS PLC)<br>* claims 1, 5-8 *<br><br>* claim 9 * | <br>1, 2, 5, 7<br>9 | C07D498/18<br>C07H19/01<br>A61K31/435<br>//(C07D498/18, 311:00,273:00, 221:00) |
| D,X | EP-A-0315978 (SANDOZ AG)<br>* claims 1-10 * | 1, 5-8 | |
| D,X<br><br>D | US-A-4894366 (M. OKUHARA ET AL.)<br>* claims 1, 12 *<br>& EP-A-0184162 | 1, 5, 7 | |
| A,D | EP-A-0323042 (FISONS PLC)<br>* claims 1, 8 * | 1, 5, 7 | |
| A,D | TETRAHEDRON LETTERS,<br>vol. 29, no. 3, 1988,<br>pages 277 - 280; D. ASKIN et al.:<br>"A diastereospecific, non-racemic synthesis of the C.10-C.18 segment of FK-506" | 1 | |
| | | | TECHNICAL FIELDS SEARCHED (Int. Cl.5 ) |
| A,D | JOURNAL OF THE AMERICAN CHEMICAL SOCIETY,<br>vol. 111, 1989,<br>pages 1157 - 1159; T.K. JONES et al.:<br>"Total Synthesis of the Immunosuppressant [-]-FK-506" | 1 | C07D498/00<br>C07H19/00 |
| A | HOUBEN-WEYL: "Methoden der Organischen Chemie" 1955, Ed.: E. MÜLLER, vol. IV, part 2; G. Thieme Verlag, Stuttgart, DE<br>* pages 14, 15, paragraph b) * | 9 | |
| A | J. R. KOSAK [Editor]: "Catalysis of Organic Reactions"<br>1984, Chapter 14, pages 279-294; Marcel Dekker, Inc., New York, N.Y., US<br>* page 282, last paragraph to page 284 * | 9 | |

The present search report has been drawn up for all claims

| Place of search | Date of completion of the search | Examiner |
|---|---|---|
| BERLIN | 18 JULY 1991 | HASS C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)